# EUROPEAN PATENT SPECIFICATION

(11) **EP 1 210 914 B1**
(45) Date of publication and mention of the grant of the patent: **11.05.2005**
(21) Application number: 00951549.5
(22) Date of filing: 04.08.2000
(51) Int. Cl.: A61B 17/70

(54) **INTERVERTEBRAL FIXING SYSTEM USED IN TREATMENTS OF THE SPINAL COLUMN**
INTERVERTEBRALES FIXIERUNGSSYSTEM ZUR VERWENDUNG BEI DER BEHANDLUNG DES RÜCKGRATES
SYSTEME DE FIXATION INTERVERTEBRAL UTILISE DANS LES TRAITEMENTS DE LA COLONNE VERTEBRALE

(30) Priority: 05.08.1999 ES 9901798
(43) Date of publication of application: 05.06.2002
(73) Proprietor: TRAIBER, S.A., 43206 Reus (ES)
(72) Inventor: MARQUEZ ALVAREZ, Luis, Poligono Mas de Les Animes, 43206 Reus (ES)
(74) Representative: Carpintero Lopez, Francisco
(86) International application number: PCT/ES2000/000310
(87) International publication number: WO 2001/010317

(56) References cited:
- WO-A-98/34554
- WO-A1-98/44859
- DE-A1- 19 720 782
- US-A- 5 681 319
- US-A- 5 733 285
- US-A- 5 899 903

## Description

### OBJECT OF THE INVENTION

The present invention relates to an intervertebral fixation system particularly designed to immobilise two or more vertebrae by using bars which, suitably configured, are attached to the bone by pedicle nails.

The field of application of the invention is medicine, and particularly bone surgery, allowing to perform equally fixed, mobile fixations or linking plate fixations which laterally displace the area of fixation of the rod with respect to that of the screw.

A further object of the invention is to associate to said system means of fixation to the sacrum bone, in order to immobilise it with respect to the proximal area of the spinal column, that is, with respect to its lumbar area, in order to attain a tension-free fixation as the sacrum is of a spongier nature than the vertebrae and therefore less resistant.

### BACKGROUND OF THE INVENTION

One of the systems employed to immobilise two or more bones consists of implanting in these corresponding pedicle screws, each with a head provided with means for attaching to it a bar, which acts as a linking bridge between the various screws, so that as each screw is rigidly connected to the common bar the corresponding bones are thereby immobilised.

Within this bone fixation system exist three types: fixed, mobile and with linking plate.

In the fixed system the bar is permanently attached perpendicular to the screw. In the second case between the bar and the screw is provided a knuckle joint which allows to vary the orientation of the bar with respect to the screw. In the third type the bar and screw are related by an intermediate plate which separates them laterally, providing a substantial separation between the aforementioned elements, as in the case of WO98/44859.

The problems presented by this type of fixation mainly stem from the fact that the means for attaching the bar to the screw are from the start already attached to the screw, hindering the implantation of the screw and impairing the view of the surgeon in establishing a proper insertion of the screw in the bone.

### DESCRIPTION OF THE INVENTION

The intervertebral fixation system disclosed by the invention, which may be used with only slight modifications for both fixed and mobile fixations, as well as for systems requiring a link plate, solves the aforementioned problems in a fully satisfactory manner, allowing implantation of the screw to take place separately from the other parts which are involved with it, that is, in absence of the means of attaching the screw head nail to the screw connecting bar, so that it can be implanted in optimal conditions from the point of view of both handling and visibility.

For this purpose and more specifically, the screw involved in the system described in its fixed and mobile versions, consists of a functional combination of: a screw having a spherical head which is slightly cambered at its top, meant to be inserted in the bone; an anchor plate which can house the screw head, provided with large recesses for the bar to rest in, and a narrowing mouth having a tronco-conical shape which allows the screw head to pass and causes it to be retained with the aid of an open washer, which is narrowed as it is compressed axially onto the aforementioned tronco-conical mouth; a bushing which acts axially on said open washer and which is pressed in this sense by the bar itself, a second bushing for support and pressure on the opposite area o said bar, and finally a threaded plug which is coupled to the anchor plate mouth and through which is achieved the final fixation and tightening of the assembly. Said plug is provided on its lower part with a circular striation.

According to a further characteristic of the invention the head of the screw is provided with an axial orifice, blind and polygonal, which allows its implantation in the bone with the aid of an Allen wrench, prior to the implantation of the remaining screw mechanisms.

From this basic structure the bushing which acts on the open washer may incorporate at its end a polygonal prismatic shaped lug for coupling to the blind orifice of the head in a fixed system, or said bushing may have a hemispherical inner surface in order to establish a knuckle joint with the screw head.

If a linking plate system is used, said plate will include a housing similar to that of the anchor plate, but shorter and without recesses, receiving only the bushing which compresses the open washer, which will be threaded, while the bushing proper will be attached to the other end of the linking plate, and receives in it a pseudomorse cone which is used as a means for attaching to the link plate and as a seat for the bar, which will be finally fixed with the aid of a second bushing and a threaded plug as in the previous case.

Said pseudomorse cone extends as a short cylindrical threaded neck which is implanted in the linking plate, which after the implantation is attached by means of a pin and is integrally joined to the plate.

With the above described structure aids a device for transverse union of bars, which consists of a piece provided with a recess open on the sides, and suitable in shape and size for a implanting in it the bars to be joined, which recess is crosses by a transverse orifice of the piece itself, which is grooved and meant for implantation of the second bar, this piece further incorporating a threaded orifice which opens perpendicularly from the outside of the transverse orifice and which is meant to receive a tightening plug by which one bar is pressed against the other after their relative position is selected.

In accordance with the described structure the implantation of the screw is made simple and convenient, with a perfect visibility for the surgeon of the work area, as well as ensuring an ideal structural rigidity for the assembly, that is, a perfect immobilisation of the rod with respect to the screw, without danger of unwanted motion of the cones to be joined, with a minimal effort required of the doctor as well as a minimal time of intervention, and the further possibility of conveniently stiffening with respect to each other of the bars which are part of the system after they are correctly positioned, all of this with the particular characteristic that the multiple parts which participate in the system to achieve fixation of the various elements are supplied to the doctor in a pre-assembled state, suitably connected to each other so that the doctor need only perform the tightening operations after each is correctly placed.

In an alternative embodiment, the open washer which is axially pressed against the truncated conical opening of the anchor plate is absent and the bushing which is axially acting on the washer has a tronco-conical lower area sectioned into segments which can deform upon tightening in order to allow a previous passage of the spherical screw head and to then clip onto said head, so that it remains attached to an joined to the system.

Additionally, a complementary element is provided which allows fixation of the corresponding bar relating the sacrum to the lumbar vertebra(e) without any tension whatsoever.

More specifically, fixation to the sacrum is based on the use of a template which after fixing the bar to the lumbar area of the spine allows to set the exact point at which the screw must be implanted, as well as its orientation, although the angle of the sacrum screws need not be precise since it must adapt to the screw head to form a knuckle. For this is used an angular linking plate, shaped so that it absorbs the angle formed by the spine where the sacrum meets the lumbar vertebrae, which linking plate is provided at one of its ends with a transverse orifice for coupling of the spinal fixation bar, an orifice which has a toothed inner face to improve grip on the bar, and which is crossed by a threaded orifice for implantation f a tightening screw, preferably with an Allen type head, while on the other end of the clinking plate are established the means of attachment to the screw head.

Such fixation means comprise a housing with a tronco-conical narrowing opening, through which may pass freely the screw head so that it can be implanted in the bone prior to coupling of the linking plate, establishing inside said housing with a threaded side wall an open washer whose diameter in a relaxed state is greater than both the diameter of the screw head and the diameter of the narrowed opening of the housing, also allowing passage through it of the screw head, this assembly complemented by an externally threaded bushing which is preferably provided with Allen operation means, and the tightening of which causes an axial displacement of the washer with its ensuing strangulation on the screw head and fixation of the latter, with the bushing having a hemispherical housing as a seat for the screw head which allows a knuckle joint movement of these elements prior to the final tightening of the assembly.

The particular configuration of the linking plate and the fixation means to both the screw head and the bar linking it to the rest of the spine make this fixation free of tensions which may damage the sacrum in the area of implantation of the screw.

### DESCRIPTION OF THE DRAWINGS

The characteristics of the present invention will be better understood in view of the accompanying drawings made of a preferred embodiment of the invention, where for purposes of illustration only and as an integral part of the description the following is shown:
Figure 1.- Shows a perspective exploded view of an intervertebral fixation system for spinal treatments in accordance with the present invention, in which some of its elements are shown in a diametric cross section, according to a first embodiment where a fixed system is taught.
Figure 2.- Shows the same assembly of the previous figure duly assembled and in cross section.

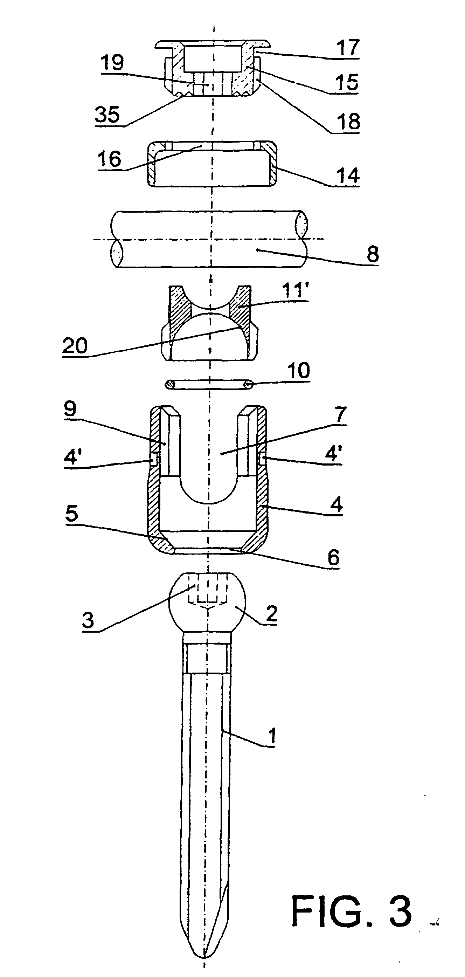

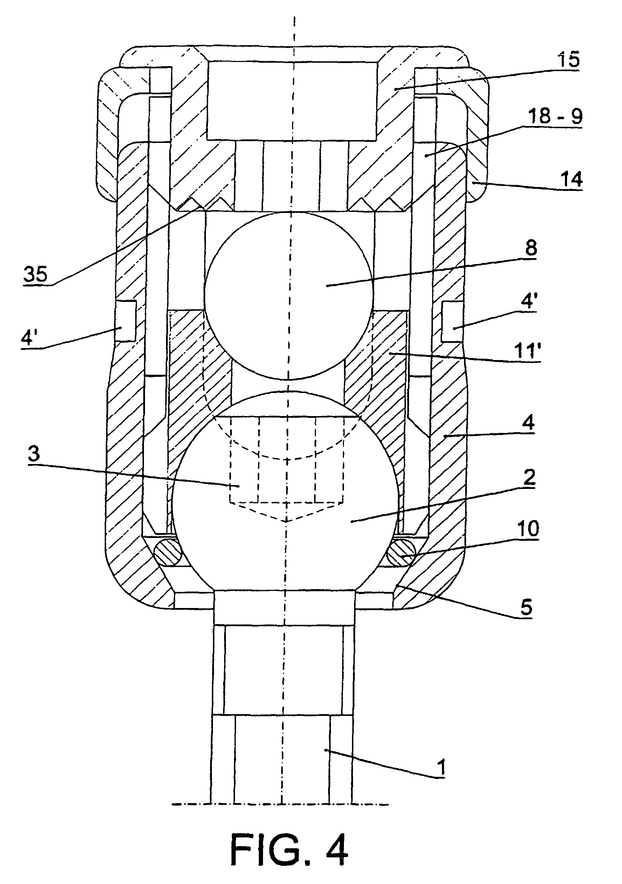

Figures 3 and 4.- Show similar representations to figures 1 and 2 corresponding here to the mobile system embodiment.
Figures 5 and 6.- Shows representations also similar to those of figures 1 and 2 here corresponding to the system which uses a linking plate.
Figure 7.- Shows a side elevation view of the device meant for transverse union of bars which is also part of the system of the invention.
Figure 8.- Shows a detail of a transverse section of the device of the previous figure along the A-B line of said figure.
Figure 9.- Shows the transverse union device fully assembled together with the element which keeps all components united, which is shown in an independent perspective view.
Figure 10.- Shows a side elevation and sectional view of the linking plate which participates in the fixation system to the sacrum bone, on which are coupled the screw head and the bar which bypasses and stiffens the sacrum to other vertebra(e) of the spine.
Figure 11.- Shows a plan view of said linking plate of the previous figure, here lacking the screw fixing it to the sacrum and the bar, as well as the latter's tightening screw.
Figure 12.- Shows a side elevation view of the bushing which presses and holds the screw head in an alternative embodiment for a mobile system.
Figure 13.- Shows a side elevation and sectional view of the bushing of the previous figure for a fixed system.
Figure 14.- Shows the assembly of the bushing of figure 12 onto the corresponding anchor plate which is part of the system object of the invention.

### PREFERRED EMBODIMENT OF THE INVENTION

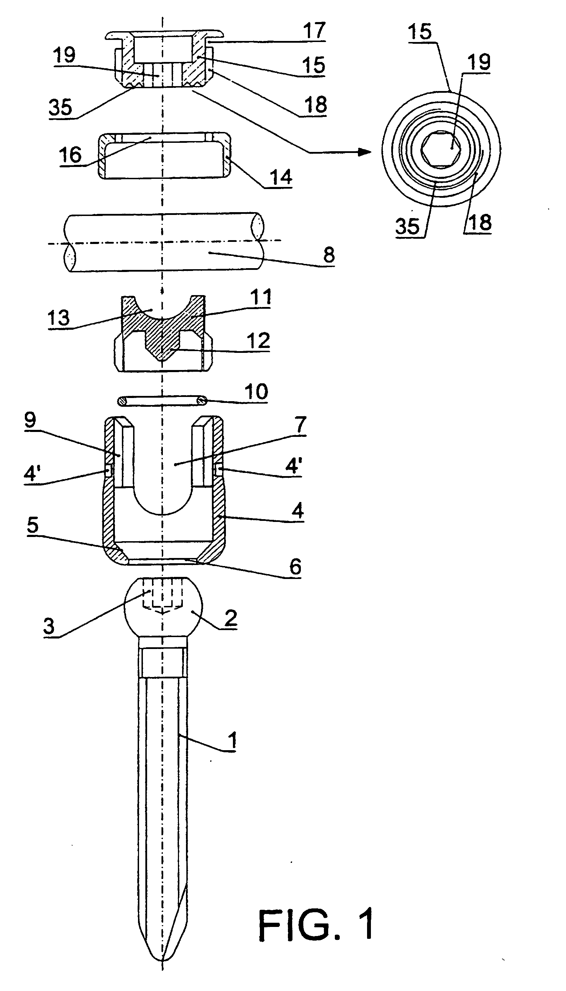

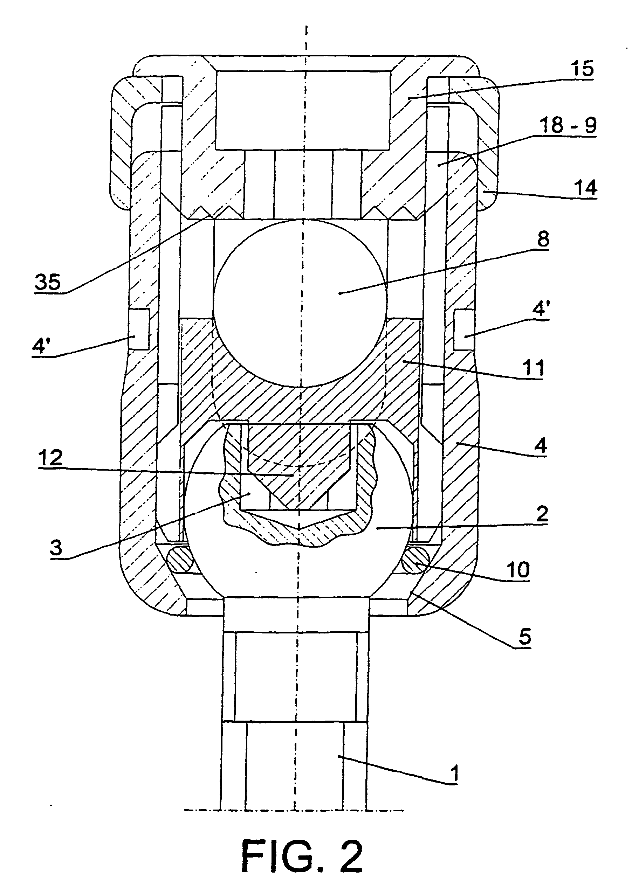

In view of these figures, and more specifically of figures 1 and 2, the intervertebral fixation system for spinal treatments disclosed by the invention comprises a screw (1), to be inserted in the bone, provided with a spherical head (2), provided with an axial blind orifice (3), with a polygonal section, to allow its implantation in the bone with the aid of an Allen wrench. Meant to be coupled to head (2) of screw (1) is an anchor plate (4) which is provided at its proximal end with respect to the screw with a tronco-conical narrowing (5), with this narrowing defining an orifice (6) which allows to pass screw head (2), and said anchor plate further incorporating large recesses (7), diametrically aligned, which allow implantation of bar (8), which is the actual piece which joins the vertebrae, as well as an inner threading (9) in the area which frames said recesses (7).

Anchor plate (4), is meant to receive within it an open washer (10) with a diameter in a relaxed state similar to the inner diameter of the lower sector of anchor plate (4), meant to narrow radially onto the lower area of screw head (2) by axial displacement of a bushing (11), which is basically cylindrical as anchor plate (4), and provided with a circular front for support on washer (10), provided with an axial lug (12), which emerges from its base and couples in blind orifice (3) of screw head (2), and through which these elements are immobilised, and further provided with an upper plate (13) where bar (8) rests.

Thus, plate (13) of bushing (11) is meant to support bar (8) and the axial pressure which is exerted through a second bushing (14) by a chreaded plug (15) coupled to threaded opening (9) of the anchor plate (4), which bushing (14) is provided at its base with a threaded orifice (16) which allows it to remain attached to plug (15), which in turn for such purpose is provided with a smooth neck (17) set back with respect to its own threaded sector (18), where bushing (14) is inserted but free. Said plug (15) also incorporates an axial, polygonal orifice (19) which can be driven by means of an Allen wrench as that used to implant screw (1). Said part (15) is provided on its lower area with a circular striation meant to ensure its grip on bar (8), which is also striated.

Anchor plate (4) is provided with a pair of lateral recesses (4') which after insertion of part (11) are recut so that said bushing (11) cannot exit nor turn, remaining positioned so that it may properly receive bar (8).

The above described structure is essentially repeated for a mobile fixation system, except that here bushing (11'), equivalent to bushing (11) of the previous case, instead of incorporating the latter's axial lug (13) incorporates a hemispherical inner surface (20) with a curvature in accordance to that of screw head (2), thereby establishing a knuckle joint between the two which allows to change the fixed situation of the previous case with bar (8) perpendicular to screw (1), so that the bar may adopt the optimal inclination with respect to the direction of the screw.

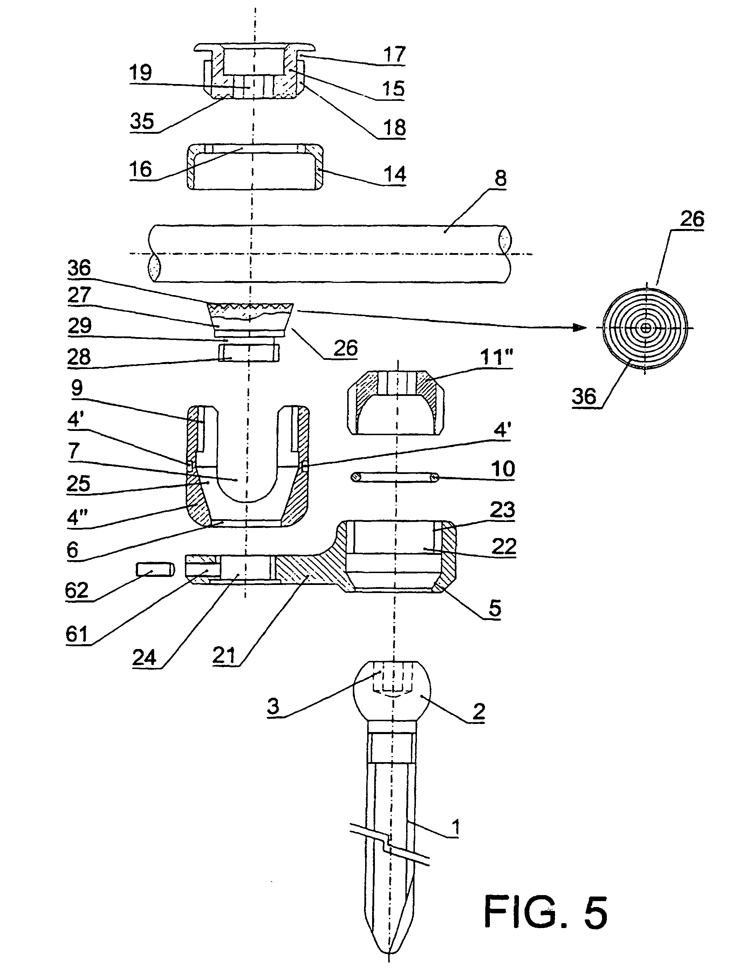

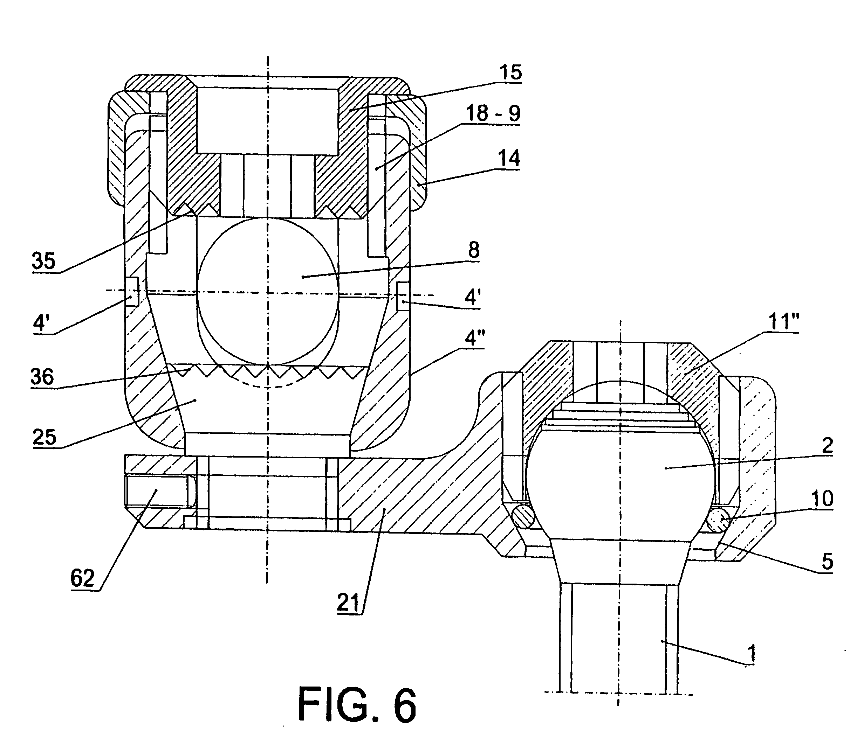

In addition and as represented in figures 5 and 6, when the system incorporates a linking plate (21) this shall be provided with a housing (22) similar to the lower sector of anchor plate (4) of the previous cases, with the same narrowing tronco-conical opening (5) and equally provided with a threading (23) for coupling of a bushing (11"), also similar to bushing (11) of the fixed system with the same axial lug (12) but with an external thread for implantation in berth (22) of plate (21), and more specifically for axial pressure on open washer (10), causing it to strangle on head (2) of screw (1).

In a different sector of lining plate (21) is provided a threaded orifice (24) for attachment to the plate of anchor plate (4"), which here configures a lower housing (25) for a pseudomorse cone (26) with a tronco-conical sector (27) and a threaded neck (28), between which is defined a neck (29) meant to immobilise pseudomorse cone (26) in order to ensure that when it is pressed on by the bar it will form a single rigid body, which will be supplied to the doctor suitably coupled to anchor plate (4"), with the free end of neck (28) riveted to prevent its decoupling from said anchor plate, but allowing a certain mobility so that bar (8) may be suitably directed, with the doctor performing the final fixation with the aid of a set screw (62) which moves in a lateral orifice (61) of plate (21), open towards orifice (24), meant to act on said neck (29) and more specifically to permanently immobilise anchor plate (4") with respect to plate (21), said cone (26) determining the seat for bar (8) which is similarly attached with the aid of bushing (14) and threaded plug (15) in sector (8) of said anchor plate (4').

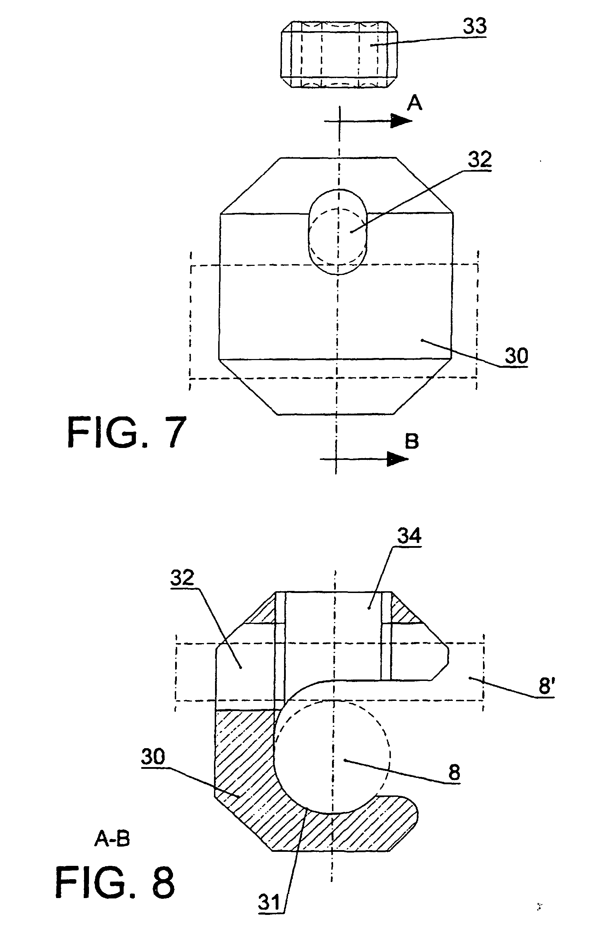

In addition to the described structure the system further includes a device for transverse union of the bars, represented in figures 7 and 8, which consists of a part (30) provided with a laterally open, concave, approximately C-shaped housing (31) for coupling and resting of one of bars (8) to be joined, which housing (31) is crossed by a transverse orifice (32), slit in the same sense as housing (31) and meant to receive the second bar (8'), with the final locking of the bars after their correct mutual positioning obtained with a threaded plug (33) which is coupled to an also threaded orifice (34) of part (30) which presses bar (8') against bar (8).

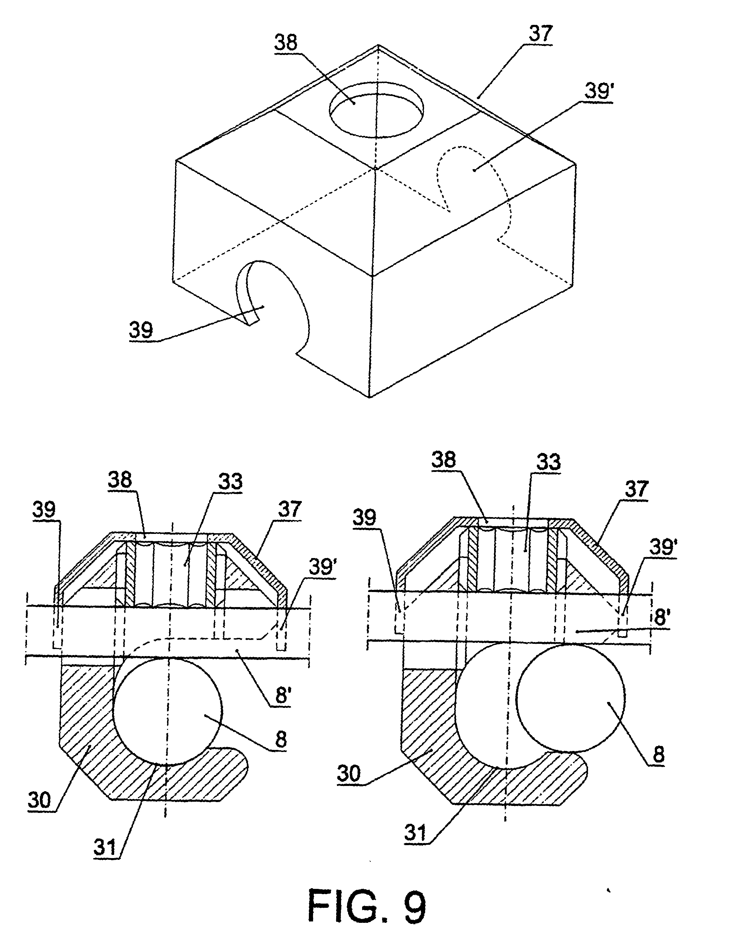

Figure 9 shows the transverse bar union device finally assembled, which comprises a cap (37) suited in shape and size to be coupled to the top of part (30) in order to prevent the latter from separating from bar (8') and retaining threaded plug (33), so that the transverse union device is mounted before or after its installation on bars (8), considerably simplifying its handling.

For this purpose cap (37) is provided on its top base with an orifice (38) through which can be accessed plug (33) so that it may be threaded, and is further provided with respective orifices (39) and (39') for passage of bar (8') through it and through transverse orifice (32). Cap (37) keeps together part (30), bar (8') and threaded plug (33), as bar (8') cannot move since it is inserted in said orifices, while cap (37) retains inside it threaded plug (33) but allows its axial displacement with respect to orifice (34) so that it may exert or release the pressure on bar (8).

To prevent part (30) and the elements joined to it from being removed through the ends of bar (8'), these ends are expanded or oversized, or any element which increases their diameter is added, characteristic which has not bee represented in the figures.

It only remains to point out that head (2) of screw (1) has striations to ensure stability of the assembly after it is tightened, and that bar (8) has a striated surface for the same purpose of providing a proper stability after it is fixed.

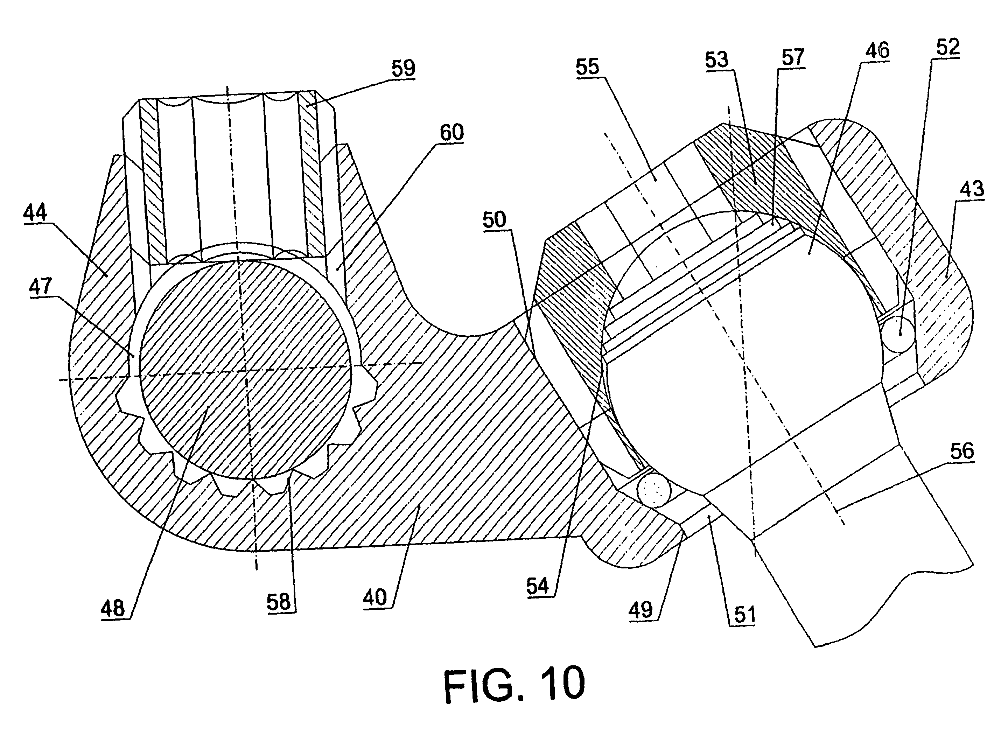

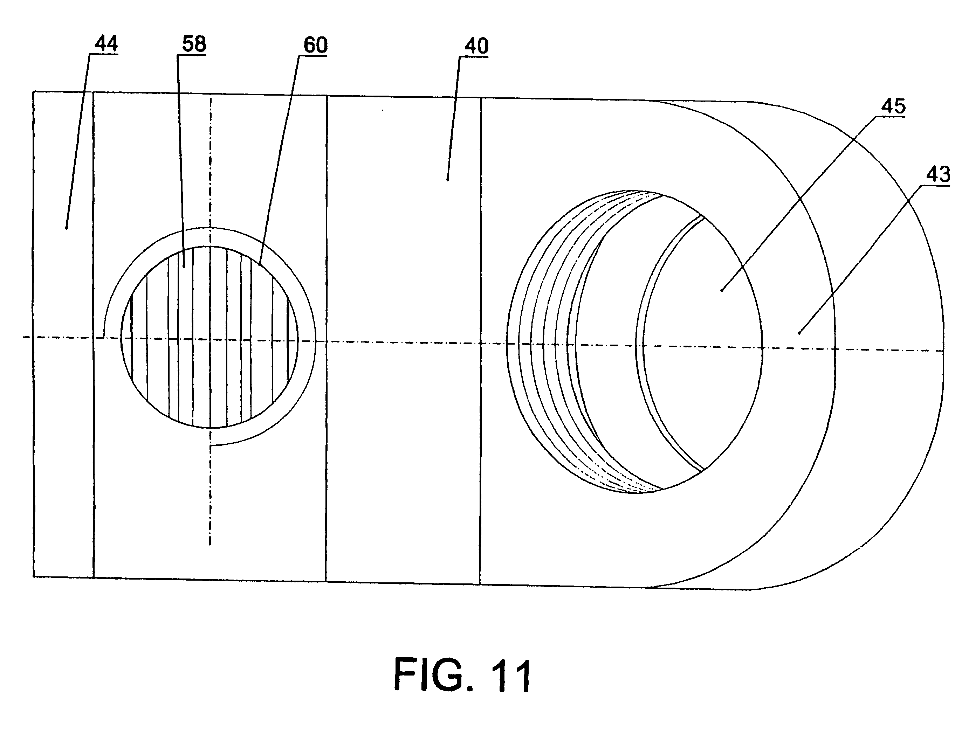

In addition to the described system, a method of attachment to the sacrum has been designed and shown in figures 10 and 11, which has as a basic element a linking plate (40) embodied as a part with two end sectors (43) and (44), the first provided with a housing (45) for head (46) of the sacrum implantation screw, and the second provided with an orifice (47) for implantation of the bar (48) meant to relate and attach the sacrum to one or more spinal vertebrae, with the particular characteristic, as seen in figure 9, that the two sectors (43) and (44) of said part form a suitable angle to compensate the angle between the sacrum and the rest of the spine.

Housing (45) for screw head (46), basically cylindrical, has a tronco-conical narrowing (49) at the end where it meets the sacrum, and a threaded sector (50) on the opposite end, with opening (51) defined by said tronco-conical narrowing (49) having a diameter greater than that of the spherical screw head (46) in order to allow the implantation of said screw in the sacrum prior to assembly on it of liking plate (40), with the truncated cone plane of narrowing (49) specifically meant to strangle an open washer (52) which rests on it, which when relaxed also has a diameter greater than that of the screw head (46) so that said head may pass through it, and which is axially displaced with the aid of a bushing (53), interiorly defining a cambered hemispherical housing (54) to allow a knuckle movement of head (46) of the screw, and which is provided with an external thread for attachment to threaded sector (50) of housing (45), and is further provided on its base with a polygonal orifice (55) for driving with an Allen wrench, all such that after screw head (46) is implanted in housing (45) and after the linking plate (40) is properly orientated with respect to axis (56) of said screw, the pressure applied by bushing (53) causes an axial displacement of washer (52) and its strangulation on the neck of the screw with the ensuing locking of the assembly, which locking is favoured by the presence of annular teeth (57) on screw head (46) which bite into the surface of housing (54) of bushing (53).

Furthermore, orifice (47) for implantation and fixation of bar (48) which links the linking plate attached to the sacrum to the adjacent area of the spine, has an inner surface provided with striations (58) meant to improve the grip on said bar, specifically when it is pressed in its housing by a screw (59) preferably with an Allen type head, which is threaded in an orifice (60) of sector (44) of linking plate (40), which opens perpendicularly towards orifice (47) for implantation of said bar.

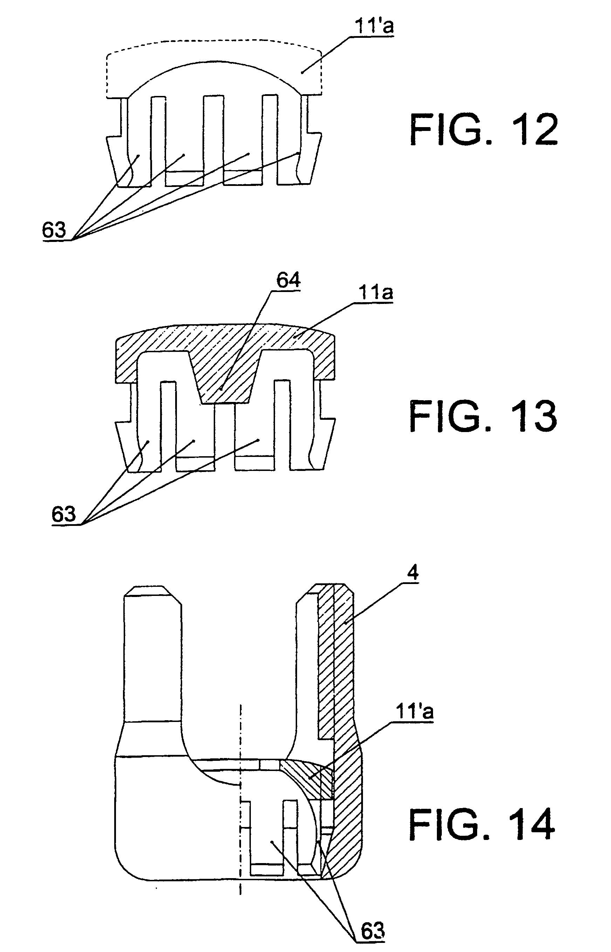

Figures 12, 13 and 14 show an alternative embodiment for bushing (11) or (11'), when the system is not provided with open washer (10).

Specifically, in this case bushing (11a) is conical at its lower area and is divided into sectors (63) which allow deformation of the part when it is tightened and meets conical area (5) of anchor plate (4), which allows entry of spherical head (2) of screw (3), and clips when pressing said bushing (11^{a}) against anchor plate (4), making the conical areas clamp spherical head (2) of screw (3), so that the latter is fixed in place and attached to the system.

As described above bushing (11a) can be used for the fixed system, while bushing (11'a) can be used in the mobile system, in which case it is provided with a central and inner appendix (64), being in all other ways including its function identical to bushing (11a).

The assembly is delivered assembled, so that its disassembly requires special clamps, first loosening the system and then, through the groove of anchor plate (4) through which the bushing projects slightly, the special clamp is used on the threads, taking care not to move the bushing and lock it when pulling on the screw, thereby allowing exit of the screw.

## Claims

1. Intervertebral fixation system for spinal treatments, which includes pedicle screws (1) having a spherical head (2) provided with a blind axial orifice (3) with a polygonal section, said screws (1) to be implanted in the bone, to which are attached bars acting as a linking means between the various screws for relative immobilisation of the corresponding bones, fixation system which may be a fixed or mobile system, and including a complement for fixation of the sacrum, the spherical head (2) of the screw (1) comprising an anchor plate (4) which in its area proximal to screw (1) is provided with a tronco-conical narrowing opening (5) which defines an orifice (6) with a diameter greater than that of screw head (2), and with said anchor plate (4) designed to house within it a bushing (11a or 11'a) on which rests bar (8) to be fixed, with an additional bushing (14) acting on the external area corresponding to bar (8), and axially pressed by a plug (15) which is threaded onto the also threaded opening (9) of anchor plate (4), which is provided with lateral recesses (4') to prevent separation or rotation of the bushing after it is inserted in anchor plate (4).

2. Intervertebral fixation system for spinal treatments, as claimed in claim 1, which for a fixed system bushing (11a) includes an inner lug (64) emerging axially from its base, with a section having the same configuration as blind orifice (3) of screw head (2) for the relative immobilisation of these two elements.

3. Intervertebral fixation system for spinal treatments, as claimed in claim 1, **characterised in that** for a mobile fixation system bushing (11'a) has a concave inner surface with a hemispherical shape, defining a knuckle type joint with head (2) of screw (1).

4. Intervertebral fixation system for spinal treatments, as claimed in previous claims, wherein the bushing is formed by a bushing (11) and an open washer (10) and the anchor plate (4) houses within it the open washer (10) and bushing (11) so that the axial displacement of the bushing (11) causes a strangulation of the washer (10) on the lower area of screw head (2) and the ensuing locking of the latter onto the anchor plate (4).

5. Intervertebral fixation system for spinal treatments, which includes pedicle screws (1) having a spherical head (2) provided with a blind axial orifice (3) with a polygonal section, said screws (1) to be implanted in the bone, to which are attached bars acting as a linking means between the various screws for relative immobilisation of the corresponding bone, fixation system which is aided by a linking plate, the linking plate (21) includes on one of its ends a housing (22) which in is area proximal to screw (1) is provided with a tronco-conical narrowing opening (5), with an inner threading (23) for receiving a bushing (11"), which is in turn provided with an external threading and which acts as a strangulation means of an open washer (10) onto the screw head, while on the other end of the linking plate (21) is defined a threaded orifice (24) in which an anchor plate (4") is fixed, which is provided with a tronco-conical housing (25) housing a pseudomorse cone (26), which is provided with a threaded neck (28) to be fixed to orifice (24) and on which cone rests bar (8), which is also fixed with the aid of an upper bushing (14) and the complementary plug (15) for axial tightening.

6. Intervertebral fixation system for spinal treatments, as claimed in claim 5, wherein a perimetral neck (29) is provided between tronco-conical sector (27) and cylindrical threaded sector (28) of pseudomorse cone (26) which neck after assembly of said cone (26) on the threaded orifice (24) of linking plate (21) is facing an also threaded lateral orifice (61) of plate (21), in which lies a pin (62) which permanently immobilises cone (26) and anchor plate (4") with respect to base plate (21).

7. Intervertebral fixation system for spinal treatments, as claimed in previous claims, wherein the lower part of plug (15) is striated to improve the grip when it is tightened, while bar (8) which connects the screws is also striated for a similar purpose.

8. Intervertebral fixation system for spinal treatments, as claimed in previous claims, wherein in it also participates a transverse bar linking device, embodied as a part (30) provided with a lateral recess (31) with a concave shape, transversally elongated with respect to its opening and meant to house on of bars (8) to be linked, said recess (31) crossed by a transverse orifice (32) which is also grooved in the same direction, meant for implantation of the second bar (8') to be joined to the first, with part (30) including a threaded orifice (34) perpendicular to both recess (31) and orifice (32), and in which is placed a threaded plug (33) for tightening one bar (8') against the other bar (8) at the point where they cross, further aiding in this a cap (37) sized and shaped so that it couples to the top of part (30), to bar (8') and to threaded plug (33) during implantation or removal of the transverse linking device, but which allows the axial displacement of threaded plug (33), for which cap (37) is provided on its top base with an orifice (38) for access to threaded plug (33), and with orifices (39 - 39') for passage of bar (8').

9. Intervertebral fixation system for spinal treatments, as claimed in previous claims, wherein the component parts are pre-assembled prior to their final assembly.

10. Intervertebral fixation system for spinal treatments, as claimed in previous claims, wherein the tightening plug (15) implies fixation of anchor plate (4) against screw (1) with the assembly forming a single unit.

11. Intervertebral fixation system for spinal treatments, as claimed in claim 4, wherein the initial fixation of screw (1) and anchor plate (4) is aided by the open washer (10) which is forced open to allow passage of the head of screw (1), and which closes firmly when said washer passes the largest diameter of the head of screw (1).

12. Intervertebral fixation system for spinal treatments, as claimed in claim 8, wherein the ends of bars (8') are widened or have attached elements which increase their diameter to prevent the their accidental exit from linking parts (30).

13. Intervertebral fixation system for spinal treatments, as claimed in claim 5, wherein the complement for fixation to the sacrum consists of a linking plate (40) meant to relate sacrum implantation screw (56) to the corresponding bar (48), adopting said linking plate an angular configuration so that between the area (43) of implantation of screw head (46) and area (44) of implantation of bar (48) is defined an angle which compensates the anatomical angle between the sacrum and the lumbar area of the spine, establishing at these ends (43) and (44) of linking plate (40) fixation means for head (46) of the screw and for bar (48).

14. Intervertebral fixation system for spinal treatments, as claimed in claim 13, wherein linking plate (40) incorporates as a means of fixation of screw head (46) a basically cylindrical housing (45) with a narrowed tronco-conical opening(49) at the height where it adapts to the sacrum, with a diameter greater than that of the screw spherical head (46), and on which tronco-conical surface (49) lies an open washer (52) which in its relaxed state has a greater diameter than the screw head to allow coupling of the linking plate to it after implanting said screw to the sacrum, and which strangles onto the neck of said screw head by action of a bushing (53) provided with a hemispherical housing (54), which defines a knuckle joint for the screw head, which bushing is threaded in the inner wall of housing (45) and is provided with an Allen type head (55) for its driving during the tightening operation.

15. Intervertebral fixation system for spinal treatments, as claimed in claims 13 and 14, wherein linking plate (40) has as fixation means to bar (48) a basically cylindrical orifice (47) through which opens radially a further orifice (60), this one threaded, in which is a screw (59), preferably Allen type, which presses bar (48) against the wall of orifice (47), which wall is provided with angular means (58) to improve grip on said bar (48).

16. Intervertebral fixation system for spinal treatments, as claimed in claims 1 to 3, wherein both bushing (11a) in the fixed system and bushing (11'a) in the mobile system are divided into sectors (63) which may deform to allow passage of head (2) of screw (1) and the later clipping of said head to hold the screw and attach it to the system.

## Patentansprüche

1. Intervertebrales Fixierungssystem für die Behandlung des Rückgrates, welches Pedikulusschrauben (1) mit einem Kugelkopf (2) einschließt, der mit einem axialen Blindloch (3) mit einem polygonalen Querschnitt versehen ist, wobei besagte Schrauben (1) im Knochen implantiert werden sollen, an denen Stangen befestigt werden, die als ein Verbindungsmittel zwischen den verschiedenen Schrauben zur relativen Immobilisierung der entsprechenden Knochen dienen, wobei das Fixierungssystem ein fixiertes oder ein mobiles System sein kann und ein Komplement zur Fixierung des Sacrums einschließt, wobei der Kugelkopf (2) der Schraube (1) eine Ankerplatte (4) umfasst, die in ihrer Fläche proximal zur Schraube (1) mit einer sich kegelstumpfartig verengenden Öffnung (5) versehen ist, die ein Loch (6) mit einem größeren Durchmesser als demjenigen des Schraubenkopfes (2) definiert, und wobei besagte Ankerplatte (4) so konstruiert ist, daß sie in sich eine Hülse (11a oder 11 'a) aufnimmt, auf der Stange (8) ruht, die mit einer weiteren Hülse (14) fixiert wird, die auf die Außenfläche, die Stange (8) entspricht, wirkt und axial von einem Stopfen (15) zusammengedrückt wird, der auf die ebenfalls mit Schraubgewinde versehene Öffnung (9) der Ankerplatte (8) aufgeschraubt wird, die mit Seitenausnehmungen (4') versehen ist, um eine Trennung oder Drehung der Hülse zu verhindern, nachdem sie in die Ankerplatte (4) eingesetzt ist.

2. Intervertebrales Fixierungssystem zur Behandlung des Rückgrates nach Anspruch 1, **dadurch gekennzeichnet, daß** für ein fixiertes System, die Hülse (11a) eine innere Nase (64) einschließt, die axial von ihrer Basis hervorsteht, mit einem Querschnitt, der dieselbe Konfiguration hat wie das Blindloch (3) des Schraubenkopfes (2) zur relativen Immobilisierung dieser zwei Elemente.

3. Intervertebrales Fixierungssystem zur Behandlung des Rückgrates nach Anspruch 1, **dadurch gekennzeichnet, daß**, für ein mobiles Fixierungssystem, die Hülse (11'a) eine konkave Innenfläche in einer Halbkugelform aufweist, die mit dem Kopf (2) der Schraube (1) eine Gelenkverbindung definiert.

4. Intervertebrales Fixierungssystem zur Behandlung des Rückgrates nach einem der vorangehenden Ansprüche, **dadurch gekennzeichnet, daß** die Hülse gebildet wird von einer Hülse (11) und einem offenen Ring (10) und die Ankerplatte (4) in sich den offenen Ring (10) und die Hülse (11) aufnimmt, so daß die axiale Verschiebung der Hülse (11) eine Einschnürung des Ringes (10) auf der unteren Fläche des Schraubenkopfes (2) und die sich daraus ergebende Verriegelung des letzteren auf der Ankerplatte (4) bewirkt.

5. Intervertebrales Fixierungssystem zur Behandlung des Rückgrates, welches Pedikulusschrauben (1) mit einem Kugelkopf (2) einschließt, der mit einem axialen Blindloch (3) mit einem polygonalen Querschnitt versehen ist, wobei besagte Schrauben (1) in den Knochen implantiert werden sollen, an denen Stangen befestigt werden, um als ein Verbindungsmittel zwischen den verschiedenen Schrauben zur relativen Immobilisierung des entsprechenden Knochens zu wirken, wobei das Fixierungssystem durch eine Verbindungsplatte unterstützt wird, wobei die Verbindungsplatte (21) an einem ihrer Enden ein Gehäuse (22) einschließt, das in seiner Fläche proximal zur Schraube (1) mit einer sich kegelstumpfförmig verengenden Öffnung (5) versehen ist, mit einem Innengewinde (23) zur Aufnahme einer Hülse (11"), die ihrerseits mit einem Außengewinde versehen ist und die als ein Einschnürungsmittel für einen offenen Ring (10) auf dem Schraubenkopf wirkt, während auf dem anderen Ende der Verbindungsplatte (21) eine mit Schraubgewinde versehene Öffnung (24) definiert ist, in der eine Ankerplatte (4") fixiert wird, die mit einem kegelstumpfförmigen Gehäuse (25) versehen ist, das einen Pseudomorsekegel (26) aufnimmt, der mit einem mit Schraubgewinde versehenen Hals (28) versehen ist, der an Öffnung (24) fixiert wird, und wobei auf diesem Kegel eine Stange (8) ruht, die auch mit Hilfe einer oberen Hülse (14) und dem komplementären Stopfen (15) zur axialen Befestigung fixiert wird.

6. Intervertebrales Fixierungssystem zur Behandlung des Rückgrates nach Anspruch 5, **dadurch gekennzeichnet, daß** ein perimetraler Hals (29) zwischen dem kegelstumpfförmigen Abschnitt (27) und dem zylindrischen mit Schraubgewinde versehenen Abschnitt (28) des Pseudomorsekegels (26) versehen ist, wobei dieser Hals nach dem Einbau besagten Kegels (26) in der mit Schraubgewinde versehenen Öffnung (24) von Verbindungsplatte (28) einem ebenfalls mit Schraubgewinde versehenen Seitenloch (61) von Platte (21) zugewandt ist, in dem ein Stift (62) liegt, der Kegel (26) und Ankerplatte (4") in Bezug auf Basisplatte (21) permanent immobilisiert.

7. Intervertebrales Fixierungssystem für die Behandlung des Rückgrates nach einem der vorangehenden Ansprüche, **dadurch gekennzeichnet, daß** der untere Teil von Stopfen (15) gefurcht ist, um den Griff zu verbessern, wenn er angezogen wird, während Stange (8), die die Schrauben verbindet, für einen ähnlichen Zweck ebenfalls gefurcht ist.

8. Intervertebrales Fixierungssystem zur Behandlung des Rückgrates, **dadurch gekennzeichnet, daß** es ebenfalls eine Querstangenverbindungseinheit umfasst, die als ein Teil (30) verkörpert ist, das mit einer Querausnehmung (31) mit einer konkaven Form versehen ist, in Querrichtung verlängert in Bezug auf seine Öffnung und dazu gedacht, eine der Stangen (8), die miteinander verbunden werden sollen, aufzunehmen, wobei besagte Ausnehmung (31) von einer Queröffnung (32) gekreuzt wird, die ebenfalls in derselben Richtung mit einer Nut versehen ist, die gedacht ist zur Implantation der zweiten Stange (8'), die mit der ersten verbunden werden soll, wobei Teil (30) eine mit Schraubgewinde versehene Öffnung (34) einschließt, die senkrecht ist sowohl zu Ausnehmung (31) als auch zu Öffnung (32) und in der ein mit Schraubgewinde versehener Stopfen (33) zum Festziehen einer Stange (8') gegen die andere Stange (8) an dem Punkt, wo sie sich kreuzen, angebracht wird, wobei hierbei weiter eine Kappe (37) hilft, die so dimensioniert und ausgeformt ist, daß sie mit dem oberen Ende des Teils (30), mit Stange (8') und mit dem mit Schraubgewinde versehenen Stopfen (33) während der Implantation oder Entfernung der Querverbindungseinheit koppelt, aber die die axiale Verschiebung des mit Schraubgewinde versehenen Stopfens (33) ermöglicht, wofür Kappe (37) auf ihrer Oberseite mit einem Loch (38) für den Zugang zu dem mit Schraubgewinde versehenen Stopfen (33) und mit Löchern (39-39') für den Durchgang von Stange (8') versehen ist.

9. Intervertebrales Fixierungssystem zur Behandlung des Rückgrates nach einem der vorangehenden Ansprüche, **dadurch gekennzeichnet, daß** die Komponententeile vor ihrem endgültigen Zusammenbau vorzusammengebaut werden.

10. Intervertebrales Fixierungssystem zur Behandlung des Rückgrates nach einem der vorangehenden Ansprüche, **dadurch gekennzeichnet, daß** der Festziehstopfen (15) die Fixierung von Ankerplatte (4) gegen Schraube (1) mit dem Zusammenbau impliziert, wobei die Baugruppe eine einzige Einheit bildet.

11. Intervertebrales Fixierungssystem für die Behandlung des Rückgrates nach Anspruch 4, **dadurch gekennzeichnet, daß** die anfängliche Fixierung von Schraube (1) und Ankerplatte (4) unterstützt wird durch den offenen Ring (10), die auf gedrückt wird, um den Durchgang des Kopfes von Schraube (1) zu ermöglichen, und die sich fest schließt, wenn besagter Ring den größten Durchmesser des Kopfes von Schraube (1) passiert.

12. Intervertebrales Fixierungssystem für die Behandlung des Rückgrates nach Anspruch 8, **dadurch gekennzeichnet, daß** die Enden der Stangen (8') verbreitert sind oder angesetzte Elemente haben, die ihren Durchmesser erhöhen, um ihr zufälliges Herausrutschen aus den Verbindungsteilen (30) zu verhindern.

13. Intervertebrales Fixierungssystem für die Behandlung des Rückgrates nach Anspruch 5, **dadurch gekennzeichnet, daß** das Komplement zur Fixierung des Sacrums aus einer Verbindungsplatte (40) besteht, die dazu gedacht ist, die Sacrumimplantationsschraube (56) mit der entsprechenden Stange (48) zu verbinden, wobei besagte Verbindungsplatte eine Winkelkonfiguration einnimmt, so daß zwischen der Fläche (43) der Implantation von Schraubenkopf (46) und der Fläche (44) der Implantation von Stange (48) ein Winkel definiert wird, der den anatomischen Winkel zwischen dem Sacrum und der Lumbalfläche des Rückgrates kompensiert, wobei an diesen Enden (43) und (44) von Verbindungsplatte (40) Fixierungsmittel für Kopf (46) der Schraube und für Stange (48) vorgesehen sind.

14. Intervertebrales Fixierungssystem für die Behandlung des Rückgrates nach Anspruch 13, **dadurch gekennzeichnet, daß** die Verbindungsplatte (40) als ein Mittel zur Fixierung von Schraubenkopf (46) ein grundsätzlich zylindrisches Gehäuse (45) mit einer verengten kegelstumpfförmigen Öffnung (49) auf der Höhe, wo sie an das Sacrum anschließt, mit einem größeren Durchmesser als demjenigen des Schraubenkugelkopfes (46) einschließt, und wobei auf der Kegelstumpffläche (49) ein offener Ring (52) aufliegt, der in seinem entspannten Zustand einen größeren Durchmesser als der Schraubenkopf aufweist, um die Kopplung der Verbindungsplatte damit zu ermöglichen, nachdem besagte Schraube in das Sacrum implantiert ist, und der auf dem Hals besagten Schraubenkopfes durch die Wirkung einer Hülse (53) zusammengedrückt wird, die mit einem halbkugelförmigen Gehäuse (54) versehen ist, die eine Gelenkverbindung für den Schraubenkopf definiert, wobei die Hülse in der Innenwand von Gehäuse (45) mit einem Schraubgewinde versehen ist und mit einem Allen-Kopf (55) versehen ist, um sie während des Festziehvorganges vorzu-treiben.

15. Intervertebrales Fixierungssystem für die Behandlung des Rückgrates nach den Ansprüchen 13 und 14, **dadurch gekennzeichnet, daß** die Verbindungsplatte (40) als Fixierungsmittel an Stange (48) eine grundsätzlich zylindrische Öffnung (47) aufweist, durch die sich radial ein weiteres Loch (60) öffnet, wobei dieses mit einem Schraubgewinde versehen ist, in dem eine Schraube (59), vorzugsweise vom Allen-Typ, sitzt, die die Stange (48) gegen die Wand von Loch (47) drückt, wobei die Wand mit Winkelmitteln (58) versehen ist, um den Griff auf besagter Stange (48) zu verbessern.

16. Intervertebrales Fixierungssystem für die Behandlung des Rückgrates nach den Ansprüchen 1 bis 3, **dadurch gekennzeichnet, daß** sowohl Hülse (11a) im fixierten System als auch Hülse (11'a) im mobilen System in Sektoren (63) unterteilt sind, die sich verformen können, um den Durchgang von Kopf (2) von Schraube (1) und das spätere Festklemmen von besagtem Kopf zu ermöglichen, um die Schraube zu halten und sie am System zu befestigen.

## Revendications

1. Système de fixation intervertébral pour les traitements de la colonne vertébrale, comprenant des vis pédiculaires (1) ayant une tête sphérique (2) dotée d'un orifice axial borgne (3) avec une section polygonale, lesdites vis (1) étant destinées à être implantées dans l'os, auxquelles sont fixées des barres servant de moyens de liaison entre les différentes vis pour l'immobilisation relative des os correspondants, système de fixation qui peut être un système fixe ou mobile et comprenant un complément pour la fixation du sacrum, la tête sphérique (2) de la vis (1) comprenant une plaque d'ancrage (4) qui, dans sa zone proximale à la vis (1) est dotée d'une ouverture étroite tronconique (5) qui définit un orifice (6) de diamètre supérieur à celui de la tête (2) de vis, et avec ladite plaque d'ancrage (4) conçue pour renfermer une bague (11 a ou 11'a) sur laquelle s'appuie la barre (8) destinée à être fixée, avec une bague supplémentaire (14) agissant sur la zone externe correspondant à la barre (8), et comprimée axialement par une vis d'obturation (15) qui est filetée sur l'ouverture (9) également filetée de la plaque d'ancrage (4), qui est dotée d'évidements latéraux (4') pour empêcher la séparation ou la rotation de la bague après son insertion dans la plaque d'ancrage (4).

2. Système de fixation intervertébral pour les traitements de la colonne vertébrale, selon la revendication 1, dans lequel, pour un système fixe, la bague (11a) comprend une patte interne (64) sortant de manière axiale de sa base, avec une partie ayant la même configuration que l'orifice borgne (3) de la tête de vis (2) pour l'immobilisation relative de ces deux éléments.

3. Système de fixation intervertébral pour les traitements de la colonne vertébrale, selon la revendication 1, **caractérisé en ce que**, pour un système de fixation mobile, la bague (11'a) possède une surface interne concave avec une forme hémisphérique, définissant une articulation de type à genouillère avec la tête (2) de la vis (1).

4. Système de fixation intervertébral pour les traitements de la colonne vertébrale, selon l'une quelconque des revendications précédentes, dans lequel la bague est formée par une bague (11) et une rondelle ouverte (10), et la plaque d'ancrage (4) renferme la rondelle ouverte (10) et la bague (11), de sorte que le déplacement axial de la bague (11) provoque le serrage de la rondelle (10) sur la zone inférieure de la tête de vis (2) et le blocage ultérieur de cette dernière vis-à-vis de la plaque d'ancrage (4).

5. Système de fixation intervertébral pour les traitements de la colonne vertébrale, qui comprend des vis pédiculaires (1) ayant une tête sphérique (2) dotée d'un orifice axial borgne (3) avec une section polygonale, lesdites vis (1) étant destinées à être implantées dans l'os, auxquelles sont fixées des barres servant de moyens de liaison entre les différentes vis pour l'immobilisation relative de l'os correspondant, système de fixation qui est assisté d'une plaque de liaison, laquelle plaque de liaison (21) comprend, sur l'une de ses extrémités, un logement (22) qui, dans sa zone proximale à la vis (1), est doté d'une ouverture étroite tronconique (5), avec un filetage interne (23) pour recevoir une bague (11") qui est à son tour dotée d'un filetage externe et qui sert de moyens de serrage d'une rondelle ouverte (10) sur la tête de vis, alors que, sur l'autre extrémité de la plaque de liaison (21), est prévu un orifice fileté (24) dans lequel est fixée une plaque d'ancrage (4") qui est dotée d'un logement tronconique (25) recevant un cône pseudo-Morse (26) qui est prévu avec un col fileté (28) destiné à être fixé à l'orifice (24) et sur lequel cône s'appuie la barre (8) qui est également fixée à l'aide d'une bague supérieure (14) et de la vis d'obturation complémentaire (15) pour le serrage axial.

6. Système de fixation intervertébral pour les traitements de la colonne vertébrale, selon la revendication 5, dans lequel un col périmétral (29) est prévu entre le secteur tronconique (27) et le secteur fileté cylindrique (28) du cône pseudo-Morse (26), lequel col, après assemblage dudit cône (26) sur l'orifice fileté (24) de la plaque de liaison (21), fait face à un orifice latéral (61) également fileté de la plaque (21), dans lequel se trouve une broche (62) qui immobilise en permanence le cône (26) et la plaque d'ancrage (4") par rapport à la plaque de base (21).

7. Système de fixation intervertébral pour les traitements de la colonne vertébrale, selon l'une quelconque des revendications précédentes, dans lequel la partie inférieure de la vis d'obturation (15) est striée pour améliorer la prise lorsqu'elle est serrée, alors que la barre (8) qui raccorde les vis est également striée dans le même but.

8. Système de fixation intervertébral pour les traitements de la colonne vertébrale, selon l'une quelconque des revendications précédentes, dans lequel participe également un dispositif de liaison de barre transversale, mis en oeuvre en tant que partie (30) prévue avec un évidement latéral (31) avec une forme concave, transversalement allongée par rapport à son ouverture et sensée contenir l'une des barres (8) destinées à être reliées, ledit évidement (31) étant couplé par un orifice transversal (32) qui est également rainuré dans la même direction, prévu pour l'implantation de la seconde barre (8') à relier à la première, avec la partie (30) comprenant un orifice fileté (34) perpendiculaire à la fois à l'évidement (31) et à l'orifice (32), et dans laquelle est placée une vis d'obturation filetée (33) pour serrer une barre (8') contre l'autre barre (8) au point où elles se coupent, assisté en outre en cela par un capuchon (37) dimensionné et conformé de sorte qu'il se couple au haut de la partie (30), à la barre (8') et à la vis d'obturation filetée (33) pendant l'implantation ou le retrait du dispositif de liaison transversal, mais qui permet le déplacement axial de la vis d'obturation filetée (33), pour laquelle le capuchon (37) est doté, sur sa base supérieure, d'un orifice (38) pour avoir accès à la vis d'obturation filetée (33), et d'orifices (39 - 39') pour le passage de la barre (8').

9. Système de fixation intervertébral pour les traitements de la colonne vertébrale, selon l'une quelconque des revendications précédentes, dans lequel les parties composantes sont préassemblées avant leur assemblage final.

10. Système de fixation intervertébral pour les traitements de la colonne vertébrale, selon l'une quelconque des revendications précédentes, dans lequel la vis de serrage (15) implique la fixation de la plaque d'ancrage (4) contre la vis (1) avec l'assemblage qui forme une seule unité.

11. Système de fixation intervertébral pour les traitements de la colonne vertébrale, selon la revendication 4, dans lequel la fixation initiale de la vis (1) et de la plaque d'ancrage (4) est assistée par la rondelle ouverte (10) qui est forcée ouverte pour permettre le passage de la tête de la vis (1), et qui se ferme fermement lorsque ladite rondelle passe par le plus grand diamètre de la tête de la vis (1).

12. Système de fixation intervertébral pour les traitements de la colonne vertébrale, selon la revendication 8, dans lequel les extrémités des barres (8') sont élargies ou ont des éléments fixés qui augmentent leur diamètre pour empêcher leur sortie accidentelle des parties de liaison (30).

13. Système de fixation intervertébral pour les traitements de la colonne vertébrale, selon la revendication 5, dans lequel le complément pour la fixation au sacrum se compose d'une plaque de liaison (40) prévue pour relier la vis d'implantation (56) du sacrum à la barre correspondante (48), ladite plaque de liaison adoptant une configuration angulaire de sorte qu'entre la zone (43) d'implantation de la tête de vis (46) et la zone (44) d'implantation de la barre (48) est défini un angle qui compense l'angle anatomique entre le sacrum et la zone lombaire de la colonne vertébrale, établissant au niveau de ces extrémités (43) et (44) de la plaque de liaison (40), des moyens de fixation pour la tête (46) de la vis et pour la barre (48).

14. Système de fixation intervertébral pour les traitements de la colonne vertébrale, selon la revendication 13, dans lequel la plaque de liaison (40) comprend en tant que moyens de fixation de la tête de vis (46), un logement (45) globalement cylindrique avec une ouverture étroite tronconique (49) au niveau de la hauteur à laquelle il s'adapte au sacrum, avec un diamètre supérieur à celui de la tête sphérique de vis (46), et sur laquelle zone tronconique (49) se trouve une rondelle ouverte (52) qui, dans son état au repos, a un diamètre supérieur à la tête de vis pour permettre le couplage avec la plaque de liaison après l'implantation de ladite vis dans le sacrum, et qui se serre sur le col de ladite vis par l'action d'une bague (53) prévue avec un logement hémisphérique (54), qui définit une articulation à genouillère pour la tête de vis, laquelle bague est filetée au niveau de la paroi interne du logement (45) et est prévue avec une tête (55) du type Allen pour son entraînement pendant l'opération de serrage.

15. Système de fixation intervertébral pour les traitements de la colonne vertébrale, selon les revendications 13 et 14, dans lequel la plaque de liaison (40) possède, en tant que moyens de fixation à la barre (48), un orifice globalement cylindrique (47) à travers lequel s'ouvre radialement un autre orifice (60), celui-ci fileté, dans lequel on trouve une vis (59), de préférence de type Allen, qui comprime la barre (48) contre la paroi de l'orifice (47), laquelle paroi est prévue avec des moyens angulaires (58) pour améliorer la prise sur ladite barre (48).

16. Système de fixation intervertébral pour les traitements de la colonne vertébrale, selon la revendication 1 ou 3, dans lequel à la fois la bague (11a) pour le système fixe et la bague (11'a) pour le système mobile sont divisées en secteurs (63) qui peuvent se déformer pour permettre le passage de la tête (2) de la vis (1), ces derniers bloquant ladite tête pour maintenir la vis et la fixer au système.
